# EUROPEAN PATENT APPLICATION

(11) **EP 2 583 641 A1**
(43) Date of publication of application: **24.04.2013**
(21) Application number: 13152009.0
(22) Date of filing: 05.04.2007
(51) Int. Cl.: A61F 2/24

(54) **Systems and methods for loading a prosthesis onto a minimally invasive delivery system**

(30) Priority: 10.04.2006 US 790636 P; 18.07.2006 US 488395
(62) Divisional of application: 07754796.6
(71) Applicant: CoreValve, Inc., Irvine, CA 92618 (US)
(72) Inventor: Nguyen, Than, Fountain Valley, CA 92708 (US)
(74) Representative: HOFFMANN EITLE

(57) **Abstract**

A kit for orienting leaflets of a replacement valve prosthesis (100) prior to securing the prosthesis to a delivery catheter (32), the kit comprising a frustoconical housing (34) having a tapered interior surface (60), a first open end (56), and a second open end (58), said interior surface extending from said first open end to said second open end and being configured to compress a prosthesis when the prosthesis is moved through said housing and an orienting member (38) configured to be positioned within said frustoconical housing and the prosthesis to orient the leaflets of the valve in a normal open position of the leaflets in response to blood flow through the valve.

## Description

### RELATED APPLICATIONS

This application is related to, and claims priority from, U.S. Patent Application No. 11/488,395, filed July 18, 2006, and U.S. Provisional Patent Application No. 60/790,636, filed April 10, 2006, the entireties of which are hereby incorporated by reference herein and made a part of the present disclosure.

### BACKGROUND OF THE INVENTION

### Field of the Invention

The present invention relates generally to a system and method for loading a prosthetic cardiac valve assembly onto a minimally invasive delivery system, such as a delivery catheter, for example. The invention may also be used to load other nonvalvular prosthetic frames onto a delivery system.

### Description of the Related Art

Currently, the replacement of a deficient cardiac valve is often performed by opening the thorax, placing the patient under extracorporeal circulation or peripheral aorto-venous heart assistance, temporarily stopping the heart, surgically opening the heart, excising the deficient valve, and then implanting a prosthetic valve in its place. This procedure has the disadvantage of requiring prolonged patient hospitalization, as well as extensive and often painful recovery. It also presents advanced complexities and significant costs.

To address the risks associated with open-heart implantation, devices and methods for replacing a cardiac valve by a less invasive means have been contemplated. For example, it has been proposed to attach a prosthetic valve onto a support structure, in the form of a wire or network of wires, and to deliver the prosthesis transluminally using a delivery catheter.

While it is known to load an expandable stent onto a delivery catheter and deliver the stent using the delivery catheter, such systems are not readily adaptable for use with prosthetic valve assemblies. For example, present systems for loading expandable stents onto a delivery catheter are prone to damaging the valve portion of the prosthetic valve assembly when used in connection with a prosthetic valve assembly. Accordingly, a need exists for a suitable system and method of loading a prosthetic valve onto a delivery system, such as a delivery catheter, for example.

### SUMMARY OF THE INVENTION

The present invention relates generally to the loading of a transluminally implantable prosthetic valve onto a delivery catheter, or other delivery system, for a minimally invasive implantation of the prosthesis into the vasculature at a location remote from the implantation size. Preferred embodiments of the present invention preferably are used with a self-expanding prosthesis, but may also be useful in connection with balloon-expandable or other mechanically-expanded prostheses. Desirably, preferred embodiments of the present invention permit the reduction of an external dimension of a compressible valve, prosthesis without damaging the valve. Preferably, the system and method properly orient the valve relative to the frame of the prosthesis prior to or during reduction of the external dimension.

A preferred embodiment is an apparatus for reducing an external dimension of a compressible valve prosthesis including a first reducing member and a second reducing member. The first reducing member includes a first tapered surface and a first open end. The first reducing member is configured to reduce the external dimension of at least a portion of the prosthesis when the prosthesis is moved along the first tapered surface. The second reducing member includes a second tapered surface and a first open end. The second reducing member is configured to reduce the external dimension of at least a portion of the prosthesis when the prosthesis is moved along the second tapered surface.

Another preferred embodiment is a kit for orienting leaflets of a replacement valve prosthesis prior to securing the prosthesis to a delivery catheter. The kit includes a frustoconical housing having a first open end. The housing is configured to compress a prosthesis when the prosthesis is moved through the housing. The kit also includes an orienting member configured to be positioned within the frustoconical housing and the prosthesis to orient the leaflets of the valve in an open position.

A preferred method includes compressing a replacement valve prosthesis including the step of moving the prosthesis along a tapered surface in a direction wherein an inlet of the valve leads an outlet of the valve such that the prosthesis is compressed by the tapered surface.

Another preferred method of compressing a replacement valve prosthesis includes moving the prosthesis along a first tapered surface in a direction such that an outlet of the valve leads an inlet of the valve to compress at least a portion of the prosthesis. The method also includes moving the prosthesis along a second tapered surface in a direction such that the inlet of the valve leads the outlet of the valve to compress at least a portion of the prosthesis.

Yet another preferred method of compressing a prosthesis, which includes a frame supporting a valve, includes using a substantially planar contact area of a surface to apply a pushing force to an end of the prosthesis to move the prosthesis along a conical surface such that an external dimension of at least a portion of the prosthesis is compressed. The substantially planar contact area of the surface is substantially transverse to a longitudinal axis of the conical surface.

### BRIEF DESCRIPTION OF THE DRAWINGS

These and other features, aspects and advantages of the present invention are described in greater detail below in connection with drawings of a preferred system and method, which is intended to illustrate, but not to limit, the present invention. The drawings contain 26 Figures.

Figure 1 is a loading system having certain features, aspects, and advantages of the present invention. The illustrated system includes an inflow cone, an inflow tube, an outflow tube, a cap, and an outflow cone. The system is configured to facilitate the loading of a valve prosthesis (not shown) onto a delivery system, such as the illustrated delivery catheter.

Figure 2 is a perspective view of the outflow cone of the loading system of Figure 1.

Figure 3 is a cross-sectional view of the outflow cone of Figure 2, taken along view line 3-3 of Figure 2.

Figure 4 is a perspective view of the cap of the loading system of Figure 1. The cap preferably is configured to be releasably engaged to the larger of the two ends of the outflow cone.

Figure 5 is a cross-sectional view of the cap of Figure 4, taken along view line 5-5 of Figure 4.

Figure 6 is a longitudinal cross-sectional view of the inflow tube of the loading system of Figure 1.

Figure 7 is a radial cross-sectional view of the inflow tube of Figure 6, taken along view line 7-7 of Figure 6.

Figure 8 is a longitudinal cross-sectional view of the outflow tube of the loading system of Figure 1.

Figure 9 is an axial cross-sectional view of the outflow tube of Figure 8, taken along view line 9-9 of Figure 8.

Figure 10 is a perspective view of the inflow cone of the loading system of Figure 1.

Figure 11 is a cross-sectional view of the inflow cone of Figure 10, taken along view line 11-11 of Figure 10.

Figures 12-26 illustrates a preferred method of loading a valve prosthesis onto a minimally invasive delivery system, such as the illustrated delivery catheter. Figure 12 illustrates the outflow end of the valve prosthesis positioned adjacent the large opening end of the outflow cone, prior to being introduced to the tapered space within the outflow cone.

Figure 13 illustrates the valve prosthesis positioned within the outflow cone such that at least a portion of the external dimension of the valve prosthesis is reduced.

Figure 14 illustrates the cap attached to one end of the outflow cone and, preferably, assisting in positioning the valve prosthesis within the outflow cone. In addition, a portion of the inflow tube is passed through an aperture in the cap.

Figure 15 is an enlarged view of the small opening end of the outflow cone indicated by the view line 15-15 of figure 14. In Figure 15, the inflow tube is shown initially contacting an inner surface of a frame of the valve prosthesis.

Figure 16 illustrates the inflow tube being utilized to expand, or increase the external dimension of a portion of the outflow end of the valve prosthesis which protrudes from the outflow cone.

Figure 17 illustrates an inner core of a delivery catheter being passed through an inner passage of the inflow tube. In addition, Figure 17 illustrates that the outflow tube has positioned over a sheath of the delivery catheter prior to the inner core being passed through the inflow tube.

Figure 18 illustrates the inflow tube being retracted relative to the outflow cone to allow the portion of the prosthesis protruding from the outflow cone to reduce in external diameter so as to collapse onto an end support, or coupler, of the catheter inner core.

Figure 19 illustrates the sheath of the catheter and the outflow tube being advanced over an outflow end of the valve prosthesis.

Figure 20 illustrates the outflow tube and catheter sheath being advanced further over the outflow end of the valve prosthesis relative to the position shown in Figure 19.

Figure 21 illustrates the cap and inflow tube being removed from the outflow cone. In addition, the outflow cone is moved away from the valve prosthesis, along the outflow tube and catheter, toward a proximal end of the catheter.

Figure 22 illustrates the outflow tube and delivery catheter being used to advance the valve prosthesis into a large opening end of the inflow cone.

Figure 23 illustrates the outflow tube and delivery catheter being further advanced relative to the inflow cone from the position illustrated in Figure 22 such that an external dimension of an inflow end portion of the valve prosthesis is reduced.

Figure 24 illustrates the outflow tube and delivery catheter being further advanced relative to the inflow cone from the position illustrated in Figure 23, preferably to reduce the external dimension of substantially the entire portion of the valve prosthesis that is external to the outflow tube and/or the sheath of the delivery catheter.

Figure 25 illustrates the valve prosthesis being retracted into the sheath of the delivery catheter after the prosthesis has been reduced by the inflow cone. Preferably, the inner core of the delivery catheter is used to move the prosthesis relative to the sheath of the delivery catheter.

Figure 26 illustrates the valve prosthesis loaded into the delivery catheter. Further, the inflow cone and outflow tubes are illustrated as being moved in opposite directions away from the distal end of the delivery catheter to permit inspection of the distal end of the delivery catheter. Although not specifically illustrated, preferably, the outflow tube is subsequently removed from the delivery catheter.

### DETAILED DESCRIPTION OF THE PREFERRED EMBODIMENT

Although there has been considerable development and refinement of vascular stent concepts in relationship to the coronary vasculature for the treatment of myocardial infarction and angina, these concepts do not necessarily translate to prosthetic structures involving larger sections of vasculature, and more specifically, implants incorporating prosthetic valves for minimally invasive delivery from peripheral access sites of the body. For example, although a small delivery profile, or small cross-sectional configuration, is desirable for both coronary stents and prosthesis valves, the expanded size and the implantation location of prosthesis valves may introduce difficulties into the loading of a prosthesis onto a catheter or other minimally invasive delivery system.

In particular, a valve prosthesis typically requires significant reduction of the expanded external dimension in order to be loaded onto a delivery device, such as a catheter. In contrast, a typical coronary stent need only be compressed a few millimeters in size to reach its delivery configuration. Furthermore, it is necessary to avoid damaging the valve, and especially the valve leaflets, of a valve prosthesis during the loading procedure, wherein no such concern exists with a typical coronary stent. Accordingly, preferred embodiments of the present invention are well suited for use in loading an implantable prosthesis which includes a valve. However, the present loading system and method may also be used in connection with, or adapted for use in connection with, non-valvular implants, such as coronary or other types of stents, for example.

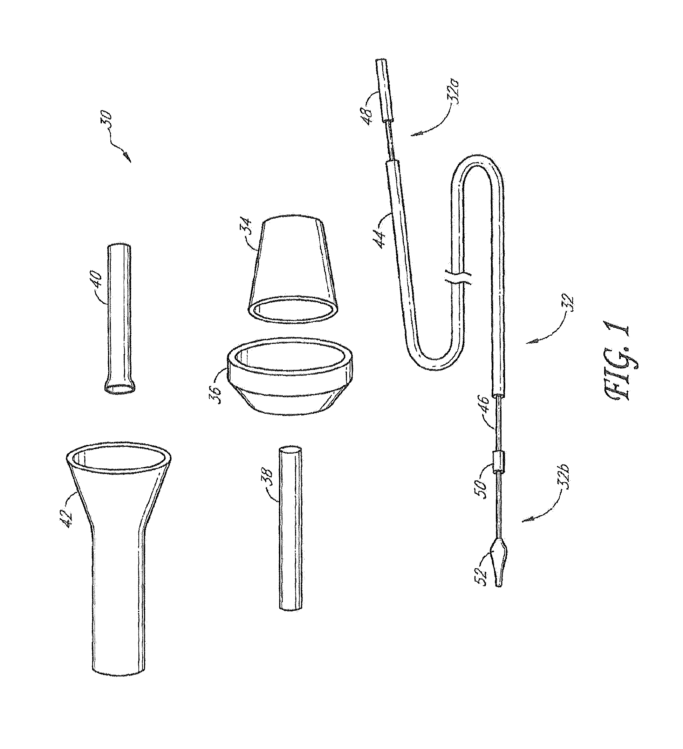

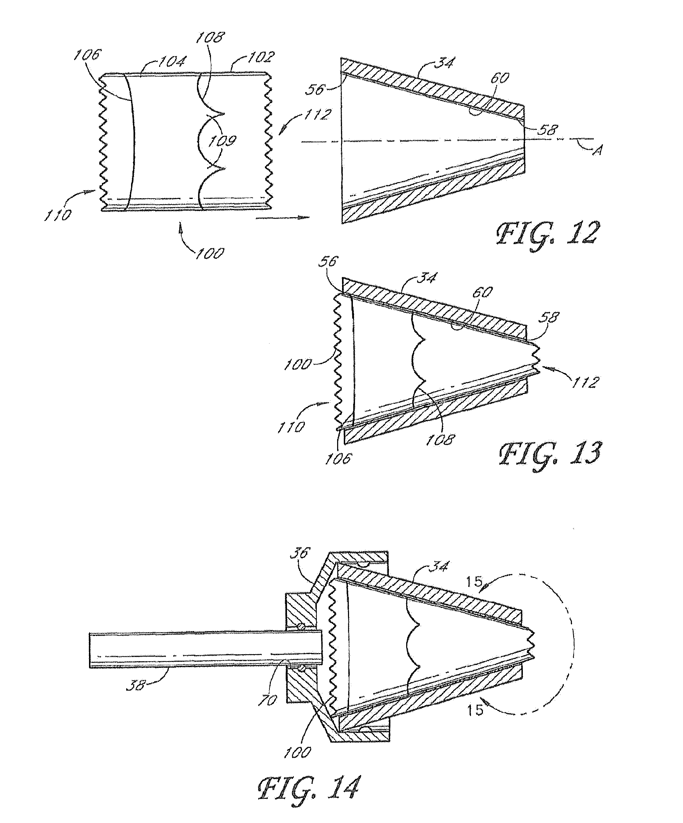

Figure 1 illustrates a loading system 30, which incorporates certain features, aspects, and advantages of the present invention. The loading system 30 is configured to facilitate the loading of a valve prosthesis (Figure 12) onto a suitable, and preferably, minimally invasive, delivery device, such as a delivery catheter 32. Because the system 30 is especially well-suited for use in loading a prosthesis that incorporates a valve, certain components of the system 30 are referred to herein using relative terminology to that components' relationship to the valve. That is, certain components of the system 30 are named or otherwise described with respect to their position relative to than inflow end or an outflow end of the valve of the implantable prosthesis. Generally, the components of the system 30 are referred to by which end of the valve prosthesis, either inflow or outflow, the component approaches the prosthesis during the illustrated loading procedure. However, the relative terminology used herein is employed as a matter of convenience for the reader and is not intended as a limitation on the present invention, unless specifically recited in the appended claims. In addition, certain positions or directions of movement of components of the system 30 may be described in relation to movement relative to the catheter 32, in which a proximal end 32a of the catheter 32 is accessible external of the patient and is manipulated by user of the system 30 and a distal end 32b of the catheter is configured to support the valve prosthesis and is introduced into the patient.

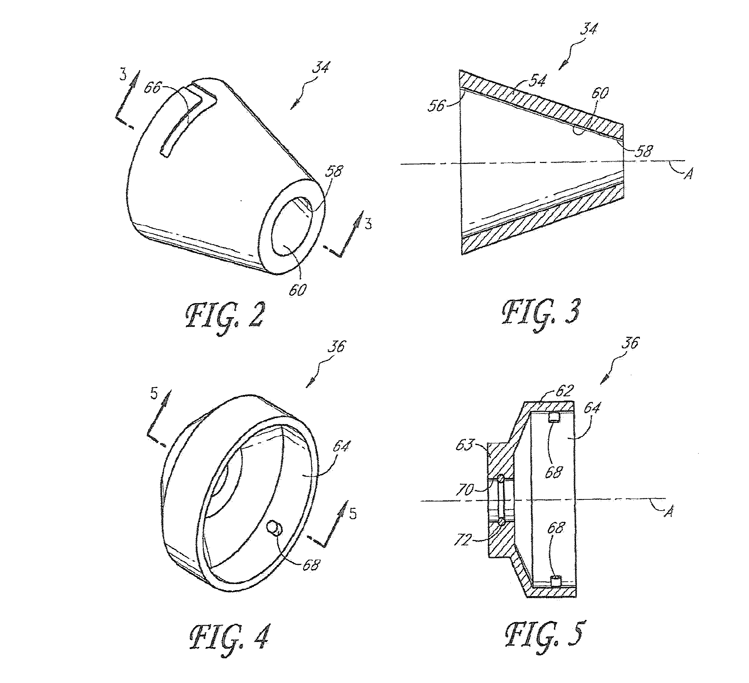

The loading system 30 preferably includes a first reducing member, or outflow cone 34. A cap 36 is releasably engageable to the large end of the outflow cone 34. An inflow tube 38 is receivable within an aperture (Figure 5) of the cap 36 and is sized to pass through a small end of the outflow cone 34, as is described in greater detail below. The system 30 preferably also includes an outflow tube 40, which is similar in structure to the inflow tube except that, preferably, one end of the outflow tube 40 includes a flare, or an enlarged diameter, as is described in greater detail below. Desirably, the system 30 also includes a second reducing member, or an inflow cone 42.

As described above, preferably the system 30 is configured to facilitate the loading of a valve prosthesis onto a delivery device and, specifically, onto the delivery catheter 32. Desirably, the delivery catheter 32 includes an outer housing, or sheath 44, and an inner core 46. The inner core 46 is received within the sheath 44 and is movable relative to the sheath 44. A proximal end of the inner core 46 may include a handle 48 to facilitate movement of the core 46 relative to the sheath 44. The handle 48 may be of any suitable style or shape to permit grasping by a user of the catheter 32.

Preferably, a distal end of the inner core 46 includes a support member, or coupler 50 and a tip 52. The support member, or coupler 50, is configured to provide internal support to an end portion of the valve prosthesis. Desirably, the support member, or coupler 50, is also configured to engage the prosthesis so that the prosthesis moves relative to the catheter sheath 44 along with movement of the inner core 46. A preferred arrangement of the coupler 50 is described in greater detail below. However, in other applications, the prosthesis may be secured another component of the catheter 32 and the coupler 50 may be omitted.

Preferably, the tip 52 is sized to substantially close off an open distal end of the catheter sheath 44 when the inner core 46 is retracted sufficiently relative to the sheath 44. In addition, preferably the tip 52 is shaped so as to be non-traumatic to tissues of the patient, including vasculature through which the catheter may travel. Thus, the tip 52 may be of any conventional or suitable shape and made from any suitable material, as will be appreciated by those of skill in the art.

With reference to Figures 2 and 3, the outflow cone 34 preferably is a generally frustoconically-shaped housing defined by a circumferential wall 54. A first end of the wall defines a first opening 56 and a second end of the wall defines a second opening 58. An inner surface 60 of the wall 54 extends between the first opening 56 and second opening 58 and defines a longitudinal axis A of the outflow cone 34.

Desirably, the first opening 56 is larger than the second opening 58 such that the surface 60 is tapered or moves closer to the axis A when moving along the surface 60 from the first opening 56 to the second opening 58. Preferably, the surface 60 is substantially linear in any plane passing through the axis A. However if desired, the surface 60 may be nonlinear, such as a stepped or curved configuration, for example. Furthermore, although the outflow cone 34 is generally circular in axial cross-section, other suitable shapes may be employed if desired.

Figures 4 and 5 illustrate a preferred embodiment of the cap 36. The cap 36 includes a circumferential wall 62 that is at least partially closed on one end by an end wall 63. The circumferential wall 62 of the cap 36 defines an internal space 64 which is sized to receive an end of the outflow cone 34 such that the cap 36 covers the opening 56 of the outflow cone 34.

As described above, preferably, the cap 36 is configured to be removably coupled to the outflow cone 34. Any suitable means of connection between the cone 34 and the cap 36 may be used. In the illustrated embodiment, an outer surface of the outflow cone 34 defines a generally J-shaped slot 66 (Figure 2). Preferably, the outflow cone 34 includes multiple slots 66- In the illustrates embodiment, the outflow cone 34 includes an opposing pair of slots 66. Each slot 66 is configured to receive a projection 68 formed on an inside surface of the circumferential wall 62 of the cap 36. Accordingly, the cap 36 may be secured to the outflow cone 34 by twisting the cap 36 relative to the cone 34. Other suitable means of connection may include corresponding threads or a frictional fit, for example, among other possibilities.

The end wall 63 of the cap 36 preferably includes an aperture 70. In the illustrated arrangement, the aperture 70 is configured to receive the inflow tube 38. Desirably, a friction member or seal 72, such as an O-ring or O-ring-type member for example, surrounds the aperture 70 and is configured to contact an outer surface of the inflow tube 38 when the inflow tube 38 is positioned within the aperture 70. Desirably, the seal 72 is configured to produce a frictional force in response to movement of the inflow tube 38 relative to the cap 36. Accordingly, once positioned, the inflow tube 38 preferably is retained in a desired position relative to the cap 36 except upon intentional movement of the inflow tube 38 by a user of the system 30. In other arrangements, the seal 72 may be omitted and the surface of the cap 36 defining the aperture 70 may provide the desired frictional force. Alternatively, movement of the inflow tube 38 relative to the cap 36 may be inhibited by other suitable means.

The wall 62 preferably defines a longitudinal axis A of the cap 36. Preferably, the space 64 and the aperture 70 are substantially centered about the axis A. Accordingly, when the cap 36 is connected to the outflow cone 34, the axes A of the outflow cone 34 and cap 36 preferably are substantially aligned.

The outflow cone 34 and cap 36 may be constructed of any suitable material or materials. For example, the outflow cone 34 and cap 36 may be constructed of materials commonly used in medical device applications and, specifically, materials used in the construction of prior stent loading devices. For example, suitable polymeric materials or metals, such as stainless steel, for example, may be used. As is discussed below, preferably, some or all of the loading steps of the valve prosthesis are performed in a cold liquid bath. Accordingly, the material(s) used for the outflow cone 34 and cap 36 preferably are relatively dimensionally stable when exposed to temperatures at or relatively near the freezing point of water, or approximately 0 degrees Celsius or 32 degrees Fahrenheit. Alternatively, the outflow cone 34 and cap 36 may be made from the same material(s) or materials that have similar coefficients of thermal expansion.

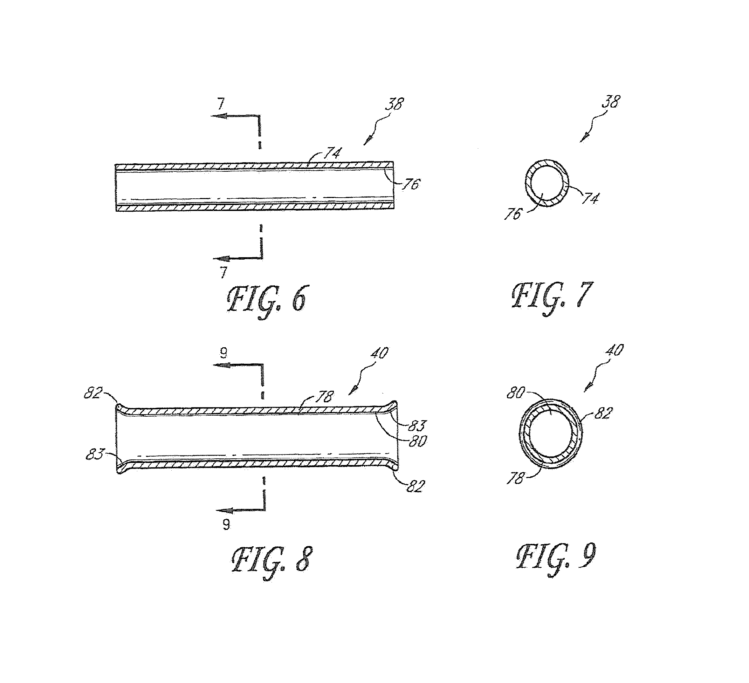

With reference to Figures 6 and 7, the inflow tube 38 preferably is an elongate tubular member. The inflow tube 38 includes a wall 74, which defines an elongate internal passage 76. Desirably, the wall 74 is generally linear in a longitudinal direction and generally circular in axial cross-section, as illustrated in Figure 7. However, other suitable shapes may also be used.

Preferably, the inflow tube 38 is made from a metal material, such as stainless steel, for example. However, other suitable material may also be used, including polymeric materials or composites, for example.

As described above, preferably an external dimension of the inflow tube 38 is sized such that the inflow tube 38 may be passed through the aperture 70 of the cap 36. Desirably, the external dimension af the inflow tube 38 is sized such that the external surface of the inflow tube 38 contacts the seal 72 of the aperture 70, which functions to secure the inflow tube 38 in a desired position relative to the cap 36. Furthermore, the external dimension of the inflow tube 38 is also sized to pass through the small end or second opening 58 of the outflow cone 34 and, preferably, pass through the second opening 58 when the valve prosthesis is present within the opening 58, as is described in greater detail below. In addition, preferably, the inner passage 76 of the inflow tube 38 is configured to accommodate the inner core 46 of the catheter 32.

With reference to Figures 8 and 9, the outflow tube 40 illustrated in greater detail. The outflow tube 40 preferably is an elongate tubular member, similar to the inflow tube 38. The outflow tube 40 includes a wall 78, which defines an elongate, internal passage 80. Desirably, the wall 78 is generally linear in a longitudinal direction and, preferably, generally circular in axial cross-section, as illustrated in Figure 9. However, other suitable shapes may also be used.

Desirably, the outflow tube 40 is constructed from a rigid material, such as a plastic material suitable for use in medical device applications. Other suitable materials may also be used, such as a metal material, e.g., stainless steel, for example. In addition, other suitable materials may also be used, including other polymeric materials and composites, for example.

Desirably, at least one end of the outflow tube 40 includes an enlarged annular portion, or a flare 82. In one preferred embodiment, both ends of the outflow tube 40 include a flare 82 so that a user does not have to orient a specific end of the outflow tube 40 relative to the catheter 32 prior to use. Desirably, the flare 82 is an enlargement of the average diameter of the wall 78 such that internal and external dimensions of the flare 82 are greater than the internal and external dimensions of the remainder of the outflow tube 40. An internal surface of the flare 82 defines a rounded "lead-in" surface 83 which is configured to assist the introduction of the prosthesis into the interior of the outflow tube 40. Advantageously, the rounded lead-in surface 83 inhibits damage to the prosthesis during the reduction in the external dimension necessary to introduce the prosthesis into the outflow tube 40 that may otherwise occur without the flare 82. However, in some arrangements, or for use with some types of prostheses, one of skill in the art may determine that the flare 82 is not necessary.

The flare 82 also limits the ability of the outflow tube 40 to pass through the inflow cone 42, as is described in greater detail below. Furthermore, preferably, the flare 82 of the outflow tube 40 is capable of passing through the second opening 58 of the outflow cone 34. In addition, the passage 80 of the outflow tube 40 preferably is configured to permit the outflow tube 40 to accommodate the catheter 32, such that the outflow tube 40 may be passed over the sheath 44 of the catheter 32.

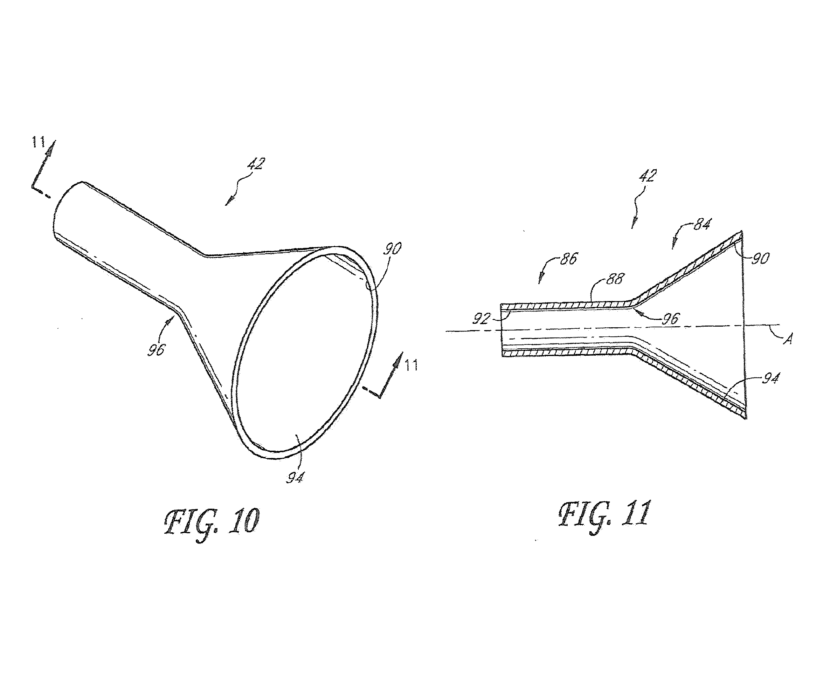

With reference to Figures 10 and 11, preferably the inflow cone 42 includes a generally elongate, hollow housing. Desirably, the inflow cone 42 includes a frustoconical portion, 84 and a tubular portion, or a substantially cylindrical portion 86. Desirably, both portions 84, 86 of the inflow cone 42 are defined by a singular wall 88. The wall 88 of the inflow cone 42 defines a first opening 90 and a second opening 92. An internal surface 94 of the wall 88 extends between the first opening 90 and the second opening 92. Desirably, the first opening 90 has a greater cross-sectional dimension than the second opening 92. In addition, preferably, the internal surface 94 at a transition portion 96 of the inflow cone 42 between the frustoconical portion 84 and the cylindrical portion 86 defines a curved transition between the inner surface 94 of each portion 84, 86. Preferably, the passage of the cylindrical portion 86 is substantially of the same diameter from the transition portion 96 to the second opening 92. The wall 88 of the inflow cone 42 defines a longitudinal axis A.

The inflow cone 42 may be constructed from any suitable material(s), such as those commonly used in medical device applications and, specifically, materials commonly utilized in stent loading applications. For example, the inflow cone 42 may be constructed of a material, or materials, similar to those used to construct the outflow cone 34 and cap 36.

Preferably, the inner diameter of the cylindrical portion 86 of the inflow cone 42 is sized such that the flare 82 of the outflow tube 40 is not able to pass through the cylindrical portion 86, but rather the flare 82 contacts the inner surface 94 of the frustoconical portion 84 at or near the transition 96. In addition, preferably, the inner diameter of the outflow tube 40 as defined by the inner surface 80 is substantially equivalent to the inner diameter of the cylindrical portion 86 of the inflow cone 42. The reasons for the desired relative dimensions of the system components 34, 36, 38, 40 and 42 discussed in the preceding paragraphs will be apparent upon review of the discussion of a preferred method of use of the system 30 with reference to Figures 12-26 below.

A preferred method of loading the valve prosthesis 100 onto the delivery catheter 32 using the loading system 30 is described with reference to Figures 12-26. Preferably, some or all of the steps illustrated and described with respect to Figures 12-26 are performed in an atmosphere that is within a range such that the frame of the valve prosthesis is in a martensite phase. Those of skill in the art will be able to determine an appropriate temperature range in which to perform the loading of other types of prostheses. In many instances, the atmosphere will be below room temperature. Desirably, all of the steps described below are performed in a cold fluid bath wherein the fluid is at or near a temperature of between about 0°C and 8°C, or between about 32°F and 46.4°F. In one arrangement, the cold fluid includes water. The water may or may not contain other substances, e.g., a saline solution. Water, or a saline solution, is preferred because it is readily available at locations in which loading of the valve prosthesis 100 may occur.

As described above, preferably, the valve prosthesis 100 includes a frame 102, which supports a valve 104, The frame 102 preferably is an elongate, hollow structure comprised of a circumferential wall that preferably is of a framework or truss-type configuration made up of a plurality of strut portions. In one embodiment, the strut portions of the frame 102 are created by the removal of material between the strut portions, such as by laser cutting, for example. In other arrangement, the frame 102 may be constructed from a wire or collection of wires.

In certain preferred embodiments, the frame 102 is constructed from a shape memory material and may be collapsed or expanded in a cross-sectional dimension. Desirably, in an expanded orientation, the frame 102 varies in cross-sectional size and/or shape along its length to assist in the collapsing of the frame 102 and/or in anchoring the prosthesis in place within a patient. In one embodiment, the frame 102 varies in radial strength along its length. For example, the end portions of the frame 102 may possess a lower radial strength than an intermediate portion of the frame 102 to assist in the collapsing of the frame 102 for loading purposes. However, the valve prosthesis 100 is illustrated in schematic fashion in Figures 12-26 and, accordingly, the desired changes in cross-sectional size and/or shape have been omitted.

The valve 104 has an inlet end 106 and an outlet end 108. The outlet end 108 preferably includes two or more cooperating valve leaflets. The inlet and outlet ends 106, 108 of the valve 104 refer to a direction of blood flow through the valve 104 when the valve prosthesis is implanted within a patient. Thus, the prosthesis 100 in general includes an inlet end 100 and an outlet end 112, which refer to the direction of blood flow through the prosthesis 100. Although the system 30 is advantageously configured for facilitating the loading of such a prosthesis 100, it will be appreciated that the system 30 may be useful with other types of implants or prosthetics as well, including prosthetics that do or do not include a valve 104. Examples of preferred arrangements of the valve prosthesis 100 are discussed in greater detail in U.S. Patent Application Nos. 10/412,634, filed April 10, 2003, and 10/772,101, filed February 4, 2004, both entitled PROSTHETIC VALVE FOR TRANSLUMINAL DELIVERY, which are assigned to the Assignee of the present application. The entirety of these applications are incorporated by reference herein and made a part of the present specification.

As illustrated in Figure 12, preferably the valve prosthesis 100 is generally aligned with the outflow cone 34 such that a longitudinal axis of the prosthesis 100 is generally aligned with the longitudinal axis A of the outflow cone 34. Preferably, the prosthesis 100 is oriented such that the outlet end 108 of the valve 104 is facing the outflow cone 34. In other words, the outlet end 108 of the valve 104 is closer to the outflow cone 34 than the inlet end 106 of the valve 104. If necessary or desired, the frame 102 of the valve prosthesis 100 may be compressed or reduced in external dimension prior to introduction into the outflow cone 34. For example, a portion of or the entire external dimension of the prosthesis 100 may be reduced by hand, or otherwise, such that the outer dimension of the prosthesis 100 is at least slightly smaller than the first opening 56 of the outflow cone 34.

The valve prosthesis 100 is moved in the direction of the arrow in Figure 12 into the outflow cone 34 through the large opening end, or first end 56. Preferably, valve prosthesis 100 is advanced within the outflow cone 34 until an outlet valve end 112 of the prosthesis 100 protrudes from the second opening 58 of the outlet cone 34. Desirably, at least a portion of the inlet end 110 of the valve prosthesis 100 is protruding from the first opening 56 of the outflow cone 34. Accordingly, the tapered inner surface 60 of the outflow cone 34 operates to reduce an external dimension of at least a portion of the prosthesis 100 as the prosthesis 100 is moved relative to the surface 60.

With reference to Figure 14, desirably the cap 36 is utilized to advance the prosthesis 100 into a desired final position within the outflow tone 34. For example, preferably the inlet end 110 of the prosthesis 100 contacts the end wall 63 (Figure 5) of the cap 36 along a circumferential or annular area of contact. Although the frame 102 of the prosthesis 100 preferably is constructed to have a relatively small wall thickness, such that the contact between the prosthesis 100 and cap 36 may be referred to as a "line" of contact, the prosthesis 100 does have some amount of wall thickness and, thus, the contact between the prosthesis 100 and cap 36 is referred to herein as a contact area. The contact area is desirably substantially planar, and is oriented substantially perpendicular to the longitudinal axis A of the outflow cone 34.

Preferably, the cap 36 is secured to outflow cone 34 and assist in maintaining a desired position of the valve prosthesis 100 within the outflow cone 34. In some arrangements, the valve prosthesis 100 may be advanced to its desired final position within the outflow cone 34 by hand. Accordingly, in such a situation, the cap 36 may not actually move the valve prosthesis 100 relative to the outflow cone 34, but may only to assist in maintaining the valve prosthesis 100 in a desired position within the outlet cone 34.

After the prosthesis 100 is positioned within the outflow cone 34 and the cap 36 is secured to the outflow cone 34, the inflow tube 38 is introduced into the aperture 70 of the cap 36, as illustrated in Figure 14. Desirably, the inflow tube 38 is advanced within the outflow cone 34 until the inflow tube 38 contacts the valve prosthesis 100 and, specifically, an inner surface of the frame 102 of the prosthesis 100. Advantageously, movement of the inflow tube 38 in this direction through the prosthesis 100 tends to properly orient the leaflets 109 of the valve 104 such that the occurrence of damage to the leaflets 109 during reduction of the prosthesis is reduced or eliminated. Thus, the inflow tube 38 preferably functions as an orienting member to orient the leaflets 109. However, in other arrangements, a separate orienting member may be used.

Preferably, the valve leaflets 109 are oriented into an open position and, more preferably, the open position is the normal orientation of the leaflets 109 when blood flow is present through the valve 104, as such an orientation has been determined to allow the leaflets 109 to collapse more evenly and lessen the likelihood of damage to the leaflets 109 when the prosthesis 100 is compressed. However, in other arrangements, the open position of the leaflets 109 may be an inverted orientation, if desired.

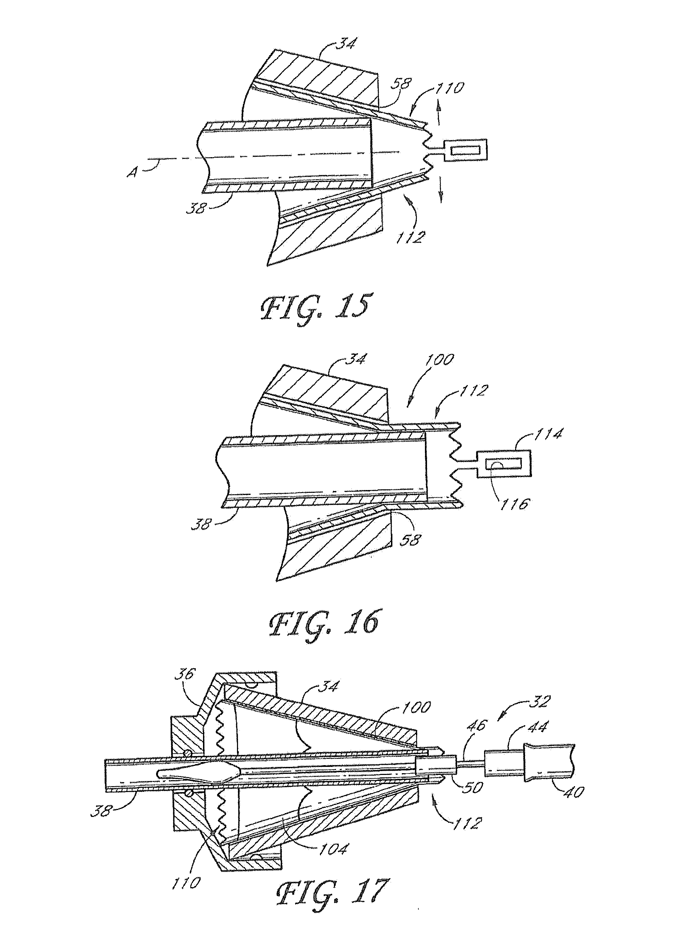

As discussed above and illustrated in Figure 15, preferably, the inflow tube 38 is sized to be able to pass through the small end, or second opening 58, of the outflow cone 34 when the valve prosthesis 100 is present within the outflow cone 34. However, desirably the inflow tube 38 has a large enough external dimension to contact the valve prosthesis 100 to enlarge or expand the outlet end 112 of the prosthesis 100 as illustrated by the arrows extending in a radial direction relative to the longitudinal axis A in Figure 15. Thus, preferably, the inflow tube 38 functions as an expansion member to expand the outlet end 112 of the prosthesis 100. In other arrangements, the expansion member may be a separate member from the inflow tube 38.

Figure 16 illustrates the outlet end 112 of the prosthesis 100 being held in an axially enlarged or expanded state by the inflow tube 38. In addition, preferably, the outflow end 112 of the prosthesis 100 includes a tab, or ear 114. Desirably, the outlet end 112 of the prosthesis 100 includes a pair of ears 114 on opposing sides of the prosthesis 100. However, in other arrangements, a greater or lesser number of ears 114 may be provided. In addition, in some arrangements, the ears 114 may be omitted and/or replaced by other suitable structures that allow the prosthesis 100 to be coupled to the delivery catheter 32 for movement in an axial direction, as it is described in greater detail below. In addition, preferably, the ear 114 defines an aperture 116.

With reference to Figure 17, preferably, the outflow tube 40 is positioned over the sheath 44 of the catheter 32. The inner core 46 of the catheter 32 is advanced from a distal end of the sheath 44 and passed through the interior of the inflow tube 38, preferably in a direction from the outlet end 112 of the prosthesis toward the inlet end 110 of the prosthesis 100, as illustrated by the arrow in Figure 17. Accordingly, once the prosthesis 100 is loaded onto the catheter 32 the prosthesis 100 will be in the appropriate orientation relative to the catheter 32 to be properly deployed within a patient, depending upon the contemplated access path. However, in other applications, such as when the prosthesis 100 does not include a valve 104 or depending on the access path, the inner core 46 of the catheter 32 may be passed through the tube 38 in the opposite direction. In addition, advantageously, because the inner core 46 of the catheter 32 is advanced through the inflow tube 38, direct contact between the inner core 46 and the valve 104 of the prosthesis 100 is eliminated such that movement of the inner core 46 does not risk undesirable reorienting of the valve leaflets 109.

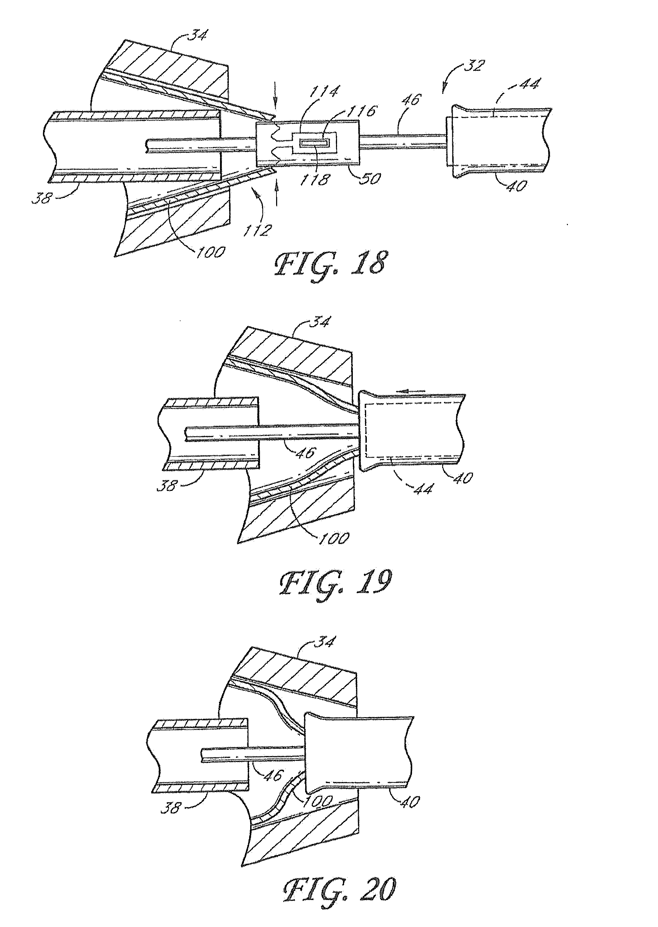

Desirably, the inner core 46 of the catheter 32 is advanced within the inflow tube 38 until the coupler 50 is positioned at or near the outflow end 112 of the prosthesis 100. With reference to Figure 18, desirably the coupler 50 includes at least one tab 118, which is configured to register with the aperture 116 of the ear 114. Preferably the coupler 50 includes a pair of tabs 118 to correspond with the pair of tabs 114 of the valve prosthesis 100 or, in other arrangements, an appropriate number of tabs 118 to correspond with the number of ears 114 provided on the prosthesis 100. The engagement of the tabs 118 into the aperture 116 of the ear 114 advantageously couples the valve prosthesis 100 for axial movement with the inner core 46 of the delivery catheter 32.

Once the inner core 46 is properly positioned relative to the prosthesis 100, the inflow tube 38 may be retracted from the outflow cone 34, in the direction indicated by the arrow in Figure 18, to allow the outflow end 112 of the valve prosthesis 100 to collapse onto the coupler 50, as illustrated by the arrows extending in a radial direction relative to the inner core 46 of the catheter 32 in Figure 18. Preferably, the inflow tube 38 is only partially retracted at this point and remains within the aperture 70 of the cap 36. In addition, because the outer surface of the inflow tube 38 preferably is smooth, retraction of the inflow tube 38 does not cause an undesired reorienting, or inversion, of the valve leaflets 109. If it is desired to unhook the valve prosthesis 100 from the coupler 50, the inflow tube 38 may be used to again expand the outlet end 112 of the prosthesis 100.

As illustrated in Figure 19, desirably, once the valve prosthesis 100 is coupled to the inner core 46 of the catheter 32, the sheath 44 of the catheter 32 and the outflow tube 40 are advanced relative to the inner core 46 toward the outlet end 112 of the valve prosthesis 100. Desirably, the sheath 44 and outflow tube 40 are advanced together over the coupler 50 and outlet end 112 of the prosthesis 100. As a result, the outflow tube 40 provides support against radial expansion of the catheter sheath 44, which may otherwise be caused by an expansion force of the prosthesis 100, to inhibit damage to the sheath 44. Preferably, care is taken to ensure that the entire circumference of the outlet end 112 of the prosthesis 100 is positioned within the sheath 44 of the catheter 32, including the ears 114.

With reference to Figure 20, desirably, the sheath 44 and outflow 240 are further advanced over the valve prosthesis 100. Desirably, the advancement of the sheath 44 and outflow tube 40 may be accomplished by retraction of the inner core 46 of the catheter 32, such as by manipulation of the handle 48 (Figure 1), for example. The sheath 44 and outflow tube 40 may be advanced up to ½ or ½ of the total longitudinal length of the valve prosthesis 100, or to a greater extent if desired. Preferably, however, the outflow tube 40 and sheath 44 are not advanced over the valve 104 of the prosthesis 100 because the outflow tube 40 and sheath 44 are moving relative to the prosthesis 100 in a direction opposite to the direction of blood flow through the valve 104. As a result of movement in the direction opposite blood flow, damage to the valve 104 or an undesirable reorienting of the leaflets 109 of the valve 104 may occur.

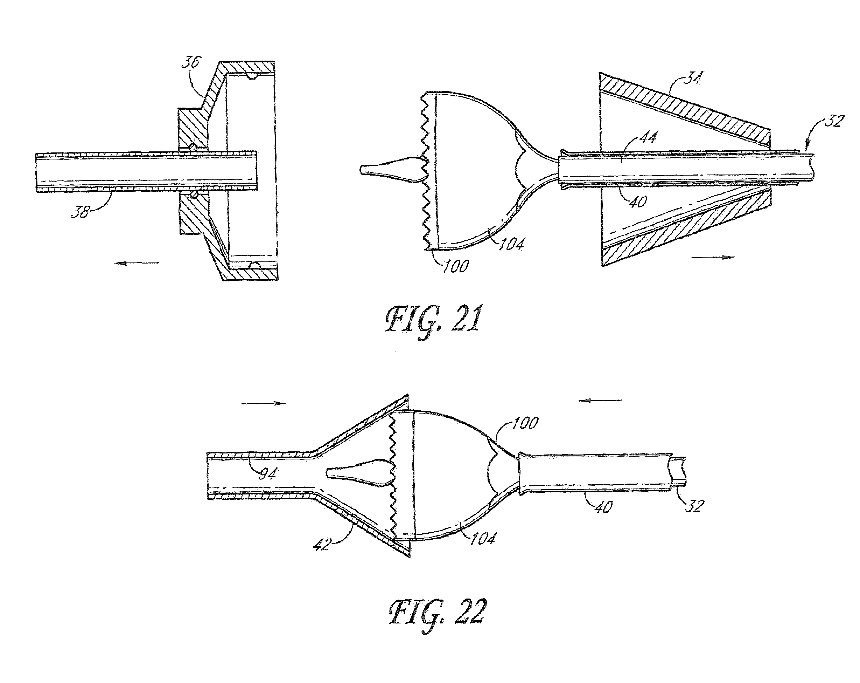

With reference to Figure 21, once the outflow tube 40 and sheath 44 have been advanced to a desired position relative to the prosthesis 100 and inner core 46 of the catheter 32, the cap 36 and inflow tube 38 may be removed from the outflow cone 34. The cap 36 and inflow tube 38 may then be withdrawn, with the inflow tube 38 being removed from over top of the inner core 46 of the catheter 32. As discussed above, desirably, because the inflow tube 38 is made from a smooth material, such as stainless steel for example, removal of the inflow tube 38 is unlikely to disturb the orientation of the leaflets 109 of the valve 104. However, once the cap and inflow tube 38 have been removed, preferably the leaflets 109 of the valve 104 are inspected to ensure that they have not been inverted or otherwise disturbed by the removal of the inflow tube 38. In addition, the outflow cone 34 may be advanced relative to the catheter 32 in a direction toward the proximal end of the catheter 32, as illustrated by the arrow in Figure 21. Accordingly, the outflow cone 34 is moved out of the way of further steps of the preferred method described below. The outflow cone 34 desirably is not removed from the distal end of the catheter 32 because it would have to pass over the prosthesis 100, a portion of which is still in a relatively expanded state.

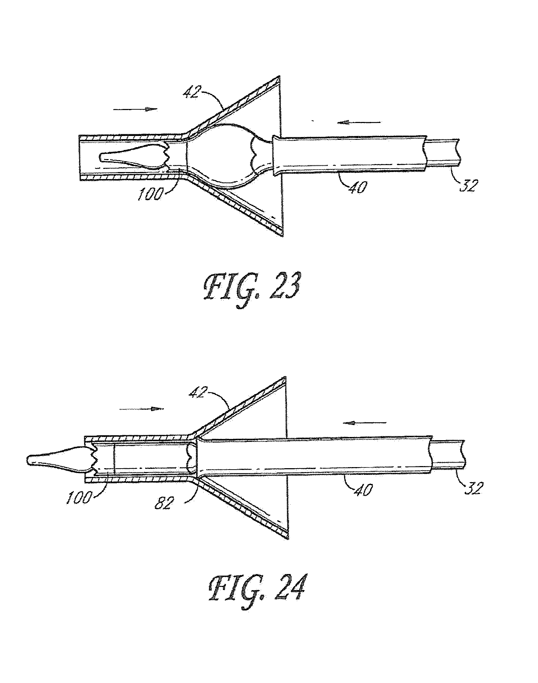

With reference to Figure 22, desirably the outflow tube 40 (and the entire catheter 32) are advanced toward the large end of the inflow cone 42. The catheter 32 and outflow tube 40 are moved toward the inflow cone 42 such that the prosthesis 100 contacts the tapered surface 94 of the inflow cone 42 and moves along the tapered surface 94 to reduce the external dimension of the prosthesis 100, as illustrated in Figures 23.

With reference to Figure 24, desirably the catheter 32 and outflow tube 40 are advanced relative to the inflow cone 42 until the end of the outflow tube 40 contacts the inner surface of the inflow cone 42. Desirably, at this point, the majority, or all, of the portion of the prosthesis 100 external to the outflow tube 40 is positioned within the cylindrical portion 86 of the inflow cone 42. Due to the enlarged outside diameter of the flare 82, in the illustrated arrangement a gap exists between the end of the outflow tube 40 and the cylindrical portion 86 of the inflow cone 42. As a result, a portion of the prosthesis 100 is not directly compressed by surfaces of either of the cylindrical portion 86 or the outflow tube 40. The gap may be minimized by making the flare 82 as small as possible while still performing its function of inhibiting damage to the prosthesis 100 during introduction into the outflow tube 40, as described above.

Advantageously, during reduction of the external dimension of the prosthesis 100 and specifically the portion of the prosthesis 100 that supports the valve 104, the prosthesis 100 is moved relative to the tapered surface 94 of the inflow cone 42 in a direction wherein the inflow end 106 of the valve 104 leads an outflow end 108 of the valve 104. Accordingly, the valve leaflets 109 tend to remain properly oriented. In addition, the base portion of the valve 104 tends to avoid becoming entrapped within the openings of the frame 102 as it is reduced or compressed.

Although in the illustrated arrangement, the prosthesis 100 is "pushed" through the tapered surface 94 of the inflow cone 42 utilizing the catheter 32, the prosthesis 100 may also be "pulled" through the inflow cone 42 utilizing a pulling member. Desirably, however, the direction that the prosthesis 100, and specifically the valve 104, moves relative to the tapered surface is as illustrated in Figures 22-24. As an additional advantage, the outflow tube 40 supports the sheath 44 of the catheter 32 against radial forces that may be produced as the prosthesis 100 is compressed to inhibit or avoid damage to the distal end of the sheath 44.

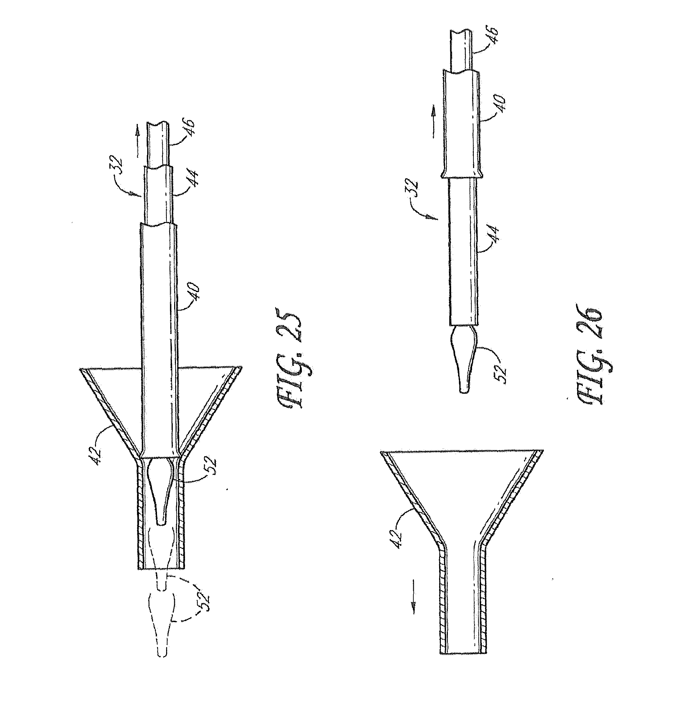

In addition, preferably, the cylindrical portion 86 of the inflow cone 42 has compressed the prosthesis 100 close or substantially to the diameter necessary for the prosthesis 100 to fit within the sheath 44 of the catheter 32. Accordingly, with reference to Figure 25, the inner core 46 of the catheter 32 may be retracted relative to the sheath 44 such that the prosthesis 100 is withdrawn into the sheath 44, as illustrated in phantom lines. Desirably, the outflow tube 40 remains over the distal end of the sheath 44 to support the sheath 44 from radial expansion due to any radial force transmitted to the sheath 44 by the prosthesis 100. In addition, although the prosthesis 100 is illustrated as being withdrawn into the sheath 44 while within the inflow cone 42, alternatively the prosthesis 100 may be removed from the cylindrical section 86 of the inflow cone 42 prior to the retraction of the inner core 46 of the catheter 32 to draw the prosthesis 100 into the sheath 44.

As illustrated in Figure 26, if it has not been so already, the inflow cone 42 may be removed from the distal end of the catheter 32 and the outflow tube 40 may be advanced along the catheter 32 toward a proximal end of the catheter to expose the distal end of the catheter. The distal end of the catheter 32 may be inspected to ensure that the prosthesis 100 is fully loaded into the sheath 44 of the catheter. Desirably, the tip 52 of the catheter abuts a distal end of the sheath 44 and substantially or fully closes off the distal end of the sheath 44. Subsequently, the outflow tube 40 and outflow cone 34 may be removed from the catheter 32.

Desirably, the prosthesis 100 is now loaded onto the delivery catheter 32 and is ready for delivery to a patient, by any suitable method. Desirably, the prosthesis 100 is positioned within the aortic annulus of a patient. Thus, with the illustrated system 30 and method, the prosthesis 100 is loaded onto the catheter 32 with the valve 104 in an appropriate orientation for delivery to the aortic annulus approaching from the aorta. In other words, the inlet end 106 (Figure 12) of the valve 104 is positioned distally on the catheter 32 from the outlet end 108 of the valve 104. However, the system 30 and/or method may be adapted for use with other applications of a prosthetic valve and with other approaches to the implantation site, as will be appreciated by those of skill in the art.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. In particular, while the present loading system and method has been described in the context of particularly preferred' embodiments, the skilled artisan will appreciate, in view of the disclosure, that certain advantages, features, and aspects of the system may be realized in a variety of other applications, many of which have been noted above. Additionally, it is contemplated that various aspects and features of the invention described can be practiced separately, combined together, or substituted for one another, and that a variety of combination and subcombinations of the features and aspects can be made and still fall within the scope of the invention. Thus, it is intended that the scope of the present invention herein disclosed should not be limited by the particular disclosed embodiments described above, but should be determined only by a fair reading of the claims.

### Further Embodiments

1. An apparatus for reducing an external dimension of a compressible valve prosthesis, said apparatus comprising:
   a first reducing member comprising a first tapered surface, said first reducing member having a first open end and configured to reduce the external dimension of at least a portion of the prosthesis when the prosthesis is moved along said first tapered surface;
   a second reducing member comprising a second tapered surface, said second reducing member having a first open end and configured to reduce the external dimension of at least a portion of the prosthesis when the prosthesis is moved along the second tapered surface.
2. The apparatus of I, wherein said first tapered surface is conical.
3. The apparatus of 2, wherein said second tapered surface is conical
4. The apparatus of 1, wherein the second reducing member further comprises a tubular portion configured to house at least a portion of the prosthesis in a reduced configuration.
5. The apparatus of 1, further comprising a releasable cap configured to cooperate with said first reducing member, said cap defining a contact surface configured to contact an end of the prosthesis to maintain the position of the prosthesis along said first tapered surface.
6. The apparatus of 5, wherein said first reducing member further comprises a second open end configured to permit an end of the prosthesis to pass through said second open end.
7. The apparatus of 6, wherein said cap includes an aperture substantially aligned with said second open end of said first reducing member, said aperture configured to receive an expansion member configured to contact an internal surface of the prosthesis when the prosthesis is positioned within the first reducing member.
8. The apparatus of 7, wherein said cap comprises a friction member configured to apply a friction force to said expansion member to resist movement of said expansion member relative to said cap.
9. The apparatus of 1, additionally comprising a containment sleeve configured to receive an end of the prosthesis.
10. The apparatus of 9, wherein said containment sleeve is also configured to support a distal end of a delivery catheter sheath which receives said end of the prosthesis.
11. An kit for orienting leaflets of a replacement valve prosthesis prior to securing the prosthesis to a delivery catheter, the kit comprising:
   a frustoconical housing having a first open end, said housing configured to compress a prosthesis when the prosthesis is moved through said housing;
   an orienting member configured to be positioned within said frustoconical housing and the prosthesis to orient the leaflets of the valve in an open position.
12. The kit of 11, wherein the open position of the leaflets is a normal position of the leaflets in response to blood flow through the valve.
13. The assembly of 11, wherein said orienting member further comprises a tube defining an interior passage configured to receive a catheter such that the catheter can be advanced through the leaflets of the valve while the leaflets are held open by said tube.
14. The assembly of 11, wherein said housing includes a second open end sized to permit an end portion of the prosthesis to pass therethrough, said housing configured to collapse the end portion of the prosthesis, said orienting member configured to be passed through the prosthesis and said second open end to bias the collapsed end portion of the prosthesis toward an expanded position.
15. The assembly of 11, further comprising a releasable cap configured to cover said first open end of said frustoconical housing, said cap defining an aperture configured to accommodate said orienting member.
16. The assembly of 15, wherein said cap further comprises a friction member configured to apply a frictional force to said orienting member to resist movement of said orienting member relative to said cap.
17. A method of compressing a replacement valve prosthesis comprising moving the prosthesis along a tapered surface in a direction wherein an inlet of the valve leads an outlet of the valve such that the prosthesis is compressed by the tapered surface.
18. The method of 17, further comprising drawing the prosthesis into a sheath of a catheter.
19. The method of 18, wherein the drawing of the prosthesis comprises moving the prosthesis in a direction such that the outlet of the valve enters the sheath before the inlet of the valve enters the sheath.
20. The method of 17, wherein the moving of the prosthesis along the tapered surface is accomplished by pushing the prosthesis.
21. The method of 17, further comprising compressing a valve outlet end of prosthesis prior to the movement of the prosthesis along the tapered surface.
22. The method of 21, further comprising positioning the valve outlet end of the prosthesis into a catheter sheath prior to the movement of the prosthesis along the tapered surface.
23. The method of 22, further comprising supporting the catheter sheath against radial expansion while moving the prosthesis along the tapered surface.
24. A method of compressing a replacement valve prosthesis, the method comprising:
   moving the prosthesis along a first tapered surface in a direction such that an outlet of the valve leads an inlet of the valve to compress at least a portion of the prosthesis;
   moving the prosthesis along a second tapered surface in a direction such that the inlet of the valve leads the outlet of the valve to compress at least a portion of the prosthesis.
25. The method of 24, further comprising utilizing a containment sleeve to restrain radial expansion of the compressed portion of the prosthesis due to the movement of the prosthesis along the second tapered surface.
26. The method of 24, wherein the moving of the prosthesis along the first tapered surface and the second tapered surface is accomplished by pushing the prosthesis.
27. The method of 24, further comprising securing the frame of the prosthesis to an inner core of a catheter and inserting a valve outlet end of the prosthesis into a sheath of the catheter after moving the prosthesis along the first tapered surface and prior to moving the prosthesis along the second tapered surface.
28. The method of 26, further comprising utilizing a containment sleeve to restrain radial expansion of a distal end of the sheath due to the movement of the prosthesis along the second tapered surface.
29. The method of 26, further comprising drawing the prosthesis into the catheter sheath after moving the prosthesis along the second tapered surface.
30. A method of compressing a prosthesis, the prosthesis including a frame supporting a valve, the method comprising using a substantially planar contact area of a surface to apply a pushing force to an end of the prosthesis to move the prosthesis along a conical surface such that an external dimension of at least a portion of the prosthesis is compressed, the substantially planar contact area of the surface being substantially transverse to a longitudinal axis of the conical surface.
31. The method of 30, further comprising securing a cap which defines the planar surface to a frustoconical housing which defines the conical surface, the frustoconical housing having an opening at a distal end configured to permit a valve outlet end of the prosthesis to extend through the opening.
32. The method of 31, further comprising inserting a tube through an aperture in the cap, the aperture generally aligned with the opening of the frustoconical housing, and expanding the valve outlet end of the prosthesis with the tube.
33. The method of 32, wherein the expanding the valve outlet end comprises contacting only the frame with the tube.

## Claims

1. A kit for orienting leaflets of a replacement valve prosthesis (100) prior to securing the prosthesis to a delivery catheter (32), the kit comprising:
a frustoconical housing (34) having a tapered interior surface (60), a first open end (56), and a second open end (58), said interior surface extending from said first open end to said second open end and being configured to compress a prosthesis when the prosthesis is moved through said housing; and
an orienting member (38) configured to be positioned within said frustoconical housing and the prosthesis to orient the leaflets of the valve in a normal open position of the leaflets in response to blood flow through the valve.

2. The kit of Claim 1, wherein said orienting member comprises a tube defining an internal passage (76) configured to receive a catheter such that the catheter can be advanced through the leaflets of the valve while the leaflets are held open by said tube.

3. The kit of Claim 1 or 2, wherein said housing is configured to collapse the end portion of the prosthesis, and said orienting member is configured to be passed through the prosthesis and said second open end to bias the collapsed end portion of the prosthesis toward an expanded position.

4. The kit of any one of Claims 1 to 3, further comprising a releasable cap (36) configured to cover said first open end of said frustoconical housing, said cap defining an aperture configured to accommodate said orienting member.

5. The kit of Claim 4, wherein said cap further comprises a friction member (72) configured to apply a frictional force to said orienting member to resist movement of said orienting member relative to said cap.
